# EUROPEAN PATENT APPLICATION

(11) **EP 4 819 152 A1**
(43) Date of publication of application: **07.10.2026**
(21) Application number: 25167740.7
(22) Date of filing: 01.04.2025
(51) Int. Cl.: G16H 30/40, G06N 3/08, G06N 20/00, G16H 50/70, A61F 5/44

(54) **METHOD AND SYSTEM FOR AUTOMATICALLY SELECTING AN OSTOMY CARE PRODUCT FROM A GIVEN SET OF OSTOMY CARE PRODUCTS**

(71) Applicant: B. Braun Avitum AG, 34212 Melsungen (DE)
(72) Inventor: MIRAMONTI, Annalisa, 34212 Melsungen (DE); ARICO, Riccardo, 64600 Anglet (FR)
(74) Representative: Patentanwälte Ruff, Wilhelm, Beier, Dauster & Partner mbB

(57) **Abstract**

Method of automatically selecting an ostomy care product from a given set of ostomy care products, the method comprising the steps:
- providing image data (4) of peristomal skin (5) of a patient having a stoma (6),
- automatically analysing the image data (4),
- automatically selecting the ostomy care product from the given set of ostomy care products based on the analysed image data (4), and
- outputting information about the selected ostomy care product.

## Description

It is the object of the invention, to provide for a method and a system for automatically selecting an ostomy care product from a given set of ostomy care products having the best suited properties for the peristomal skin of a patient having a stoma.

The method of automatically selecting an ostomy care product from a given set of ostomy care products comprises the steps: providing and/or generating digital image data of peristomal skin of a patient or user having a stoma, e.g. by means of a camera of a mobile phone, automatically analysing the image data, automatically selecting the ostomy care product from the given set of ostomy care products having the best suited properties for the peristomal skin based on the analysed image data, and outputting information to the patient or user about the selected ostomy care product. The information may be the selected ostomy care product, e.g. in form of a part number, a picture, etc., and may comprise additional details of the selected ostomy care product.

According to an embodiment the method comprises the steps: providing and/or generating an electronically generated decision tree, e.g. on a display of a mobile phone, collecting an overall patient status and information about the ostomy type by user or patient inputs electronically guided by the electronic decision tree, and automatically selecting the ostomy care product from the given set of ostomy care products based on the analysed image data, based on the overall patient status and the information about the ostomy type. The ostomy type may e.g. be colostomy, ileostomy, urostomy, etc.

According to an embodiment the ostomy care products comprised in the given set of ostomy care products differ by properties of their skin adhesives, wherein the method comprises the step: automatically selecting the ostomy care product having the best suited skin adhesive for the condition of the peristomal skin based on the analysed image data and/or based on the overall patient status and/or based on the information about the ostomy type.

According to an embodiment the step of automatically analysing the image data is based on machine learning.

According to an embodiment the step of automatically analysing the image data is based on a Large Language Model.

According to an embodiment the Large Language Model is trained based on different categories and types of peristomal skin issues and current general medical information on ostomy, in particular derived from literature, studies, Health Care Practitioners consulting, publications and clinical data.

According to an embodiment the Large Language Model is combined with Retrieval-Augmented Generation.

According to an embodiment the Retrieval-Augmented Generation is trained by adding unique combinations between conditions of the peristomal skin and corresponding best suited ostomy care products.

According to an embodiment the Large Language Model processes the collected overall patient status and the information about the ostomy type.

According to an embodiment the method comprises the steps: automatically finding appropriate recommendations regarding peristomal skin treatments based on the analysed image data and outputting the recommendations.

The system for automatically selecting an ostomy care product from a given set of ostomy care products comprises a camera, e.g. being part of a mobile phone, etc., capturing image data of peristomal skin of a patient having a stoma, processing means for automatically analysing the image data and automatically selecting the ostomy care product from the given set of ostomy care products based on the analysed image data, and outputting means for informing a user about the selected ostomy care product.

The system may be adapted to perform a method as described above.

The invention provides an Al-based tool designed to automatically analyse peristomal skin by capturing images of the skin around the stoma, guiding users/patients through a decision tree to collect further user data, and finally providing a product solution from an ostomy portfolio. The invention provides recommendations for best suited products and may predict potential future peristomal skin issues.

The Large Language Model (LLM) will be trained by adding all categories and types of peristomal skin issues assessed by healthcare professional experts. This will be a progressive training process, considering thousands of images and related data.

The Large Language Model (LLM) will receive training by incorporating current general medical information on ostomy from literature, studies, publications, clinical data, etc.

The Retrieval-Augmented Generation (RAG) will be trained by adding all specific and customized combinations between peristomal skin and ostomy care products. In the RAG, unique combinations between peristomal skin diagnostics and ostomy care products are inserted.

The ostomy care products may differ by the used adhesive. The adhesive is the component used to stick the ostomy care product or bag on the peristomal skin. According to the invention the most suitable combination between peristomal skin and adhesive type is automatically determined, consisting of a unique formulation of raw materials. Therefore, the RAG will be trained to provide a unique product solution, for example, adhesive type, to any skin condition.

The invention is in particular related to the Retrieval-Augmented Generation part. The invention will not only identify a peristomal skin issue but will also propose the most suitable combination of raw materials of the ostomy care product for the specific skin issue.

The invention offers the opportunity to automatically select the right ostomy care product not only based on feeling and ostomy type (colostomy, ileostomy, and urostomy) but also by taking into account the combination of the actual skin condition and the material composition of the ostomy care product. In other words, the invention provides a specific ostomy care product recommendation based on the skin analysis. The invention automatically determines a specific skin condition and, furthermore, recommends a corresponding specific ostomy care product.

The selection of the ostomy care product will be oriented towards the raw material combination of the ostomy care product.

An e-decision tree may allow users/patients to insert data such as name, age, location, current treatments, etc., which will be the inputs for the trained LLM and RAG.

A camera functionality may collect pictures of the peristomal skin, which will be another input for the trained LLM and RAG.

The LLM supported by the RAG may process and elaborate the response that will consist in the most suitable ostomy care product for the users/patients according to their peristomal skin condition.

The invention will now be described in detail with respect to the drawings, wherein:
- Fig. 1: highly schematically depicts a given set of ostomy care products,
- Fig. 2: highly schematically depicts components of a system for automatically selecting an ostomy care product from the given set of ostomy care products depicted in fig. 1, and
- Fig. 3: depicts an inventive machine learning architecture.

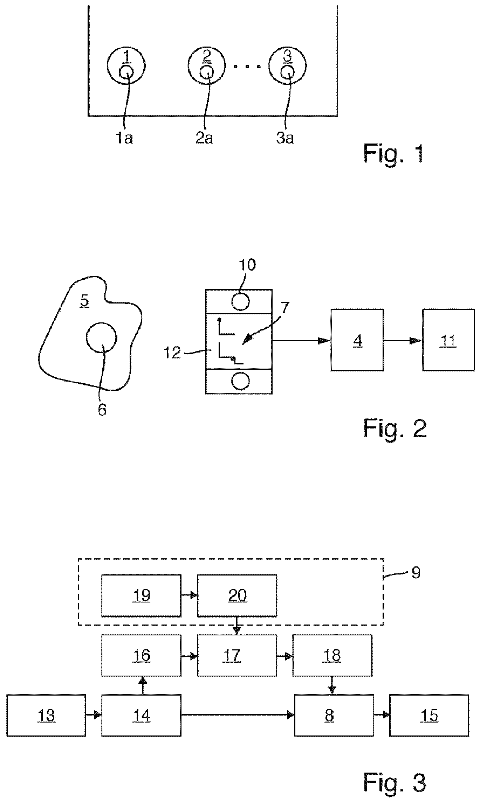

Fig. 1 highly schematically depicts a given set of ostomy care products 1, 2 and 3. Self-evidently, the set of ostomy care products 1, 2 and 3 may comprise more than the exemplarily depicted 3 ostomy care products. The following table illustrates ostomy care products that may be comprised in the set.

| Type of bags | Type of adhesive | Adhesive key features | Designed to fulfill individual needs | Tailored for specific skin injuries |
|---|---|---|---|---|
| 1-piece closed (Colostomy) | G | Rapid absorption/gentle adhesion | Secure seal | Irritation, stripping, inflammation |
| 1-piece closed (Colostomy) | Active | Gradual absorption, instant adhesion/elasticity | Adaptive comfort | Slow healing, excessive scar tissue, skin folds |
| 1-piece drainable (II-eostomy) | G | Rapid absorption/gentle adhesion | Secure seal | Irritation, stripping, inflammation |
| 1-piece drainable (II-eostomy) | Active | Gradual absorption, instant adhesion/elasticity | Adaptive comfort | Slow healing, excessive scar tissue, skin folds |
| 1-piece urostomy, high flow (Urostomy, Ileostomy) | T2 | Rapid absorption/extended adhesion | Secure seal | Maceration, irritation, inflammation |
| 2-piece (Colostomy, Ileostomy, urostomy) | T2 | Rapid absorption/extended adhesion | Secure seal | Maceration, irritation, inflammation |

Fig. 2 highly schematically depicts components of a system for automatically selecting an ostomy care product from the given set of ostomy care products 1, 2 and 3 depicted in fig. 1.

The system comprises a camera 10 capturing image data 4 of peristomal skin 5 of a patient/user having a stoma 6, processing means 11 for automatically analysing the image data 4 and automatically selecting the ostomy care product from the given set of ostomy care products 1, 2, 3 based on the analysed image data 4, and outputting means 12 for informing the patient/user about the selected ostomy care product.

The camera 10 and the outputting means 12 may e.g. be part of a mobile device, etc. The outputting means 12 may be formed by a display of the mobile device.

The display depicts an electronically generated decision tree 7. An overall patient status and information about the ostomy type is collected electronically guided by the electronic decision tree 7. The ostomy care product is selected based on the analysed image data 4, based on the overall patient status and the information about the ostomy type.

As outlined above, the ostomy care products 1, 2, 3 may differ by properties of their skin adhesives, schematically denoted as 1a, 2a, 3a in fig. 1. The ostomy care product having the best suited skin adhesive for the condition of the peristomal skin may be selected based on the analysed image data 4.

The step of automatically analysing the image data 4 and the step of selecting is based on machine learning.

Fig. 3 depicts the underlying inventive machine learning architecture.

Block 13 represents an input block enabling a user query and block 14 generates a prompt based on the user query. The generated prompt is used as an input of the LLM 8 and the result generated in the LLM is provided to an output block 15.

Block 16 represents an embedding model, block 17 represents a vector database and block 18 represents retrieved contexts fed to the LLM 8.

A Retrieval-Augmented Generation (RAG) is denoted by reference numeral 9 and contains a block 19 containing suitable combinations between ostomy care products and peristomal skin and an embedding model 20, wherein the embedding model 20 feeds the vector database 17.

The vector database 17 is additionally fed by the embedding model 16.

Regarding the generic features of LLMs, vector databases, embedding models and RAG, reference is made to the relevant technical literature.

## Claims

1. Method of automatically selecting an ostomy care product from a given set of ostomy care products (1, 2, 3), the method comprising the steps:
- providing image data (4) of peristomal skin (5) of a patient having a stoma (6),
- automatically analysing the image data (4),
- automatically selecting the ostomy care product from the given set of ostomy care products (1, 2, 3) based on the analysed image data (4), and
- outputting information about the selected ostomy care product.

2. Method according to claim 1, **characterized by** the steps:
- providing an electronically generated decision tree (7),
- collecting an overall patient status and information about the ostomy type electronically guided by the electronic decision tree (7), and
- automatically selecting the ostomy care product from the given set of ostomy care products (1, 2, 3) based on the analysed image data (4), based on the overall patient status and the information about the ostomy type.

3. Method according to one of the preceding claims, **characterized in that**
- the ostomy care products (1, 2, 3) comprised in the given set of ostomy care products (1, 2, 3) differ by properties of their skin adhesives (1a, 2a, 3a),
- wherein the method comprises the step:
- automatically selecting the ostomy care product having the best suited skin adhesive for the condition of the peristomal skin (5) based on the analysed image data (4).

4. Method according to one of the preceding claims, **characterized in that**
- the step of automatically analysing the image data is based on machine learning.

5. Method according to claim 4, **characterized in that**
- the step of automatically analysing the image data (4) is based on a Large Language Model (8).

6. Method according to claim 5, **characterized in that**
- the Large Language Model (8) is trained based on different categories and types of peristomal skin issues and current general medical information on ostomy, in particular derived from literature, studies, Health Care Practitioners consulting, publications and clinical data.

7. Method according to claim 5 or 6, **characterized in that**
- the Large Language Model (8) is combined with Retrieval-Augmented Generation (9).

8. Method according to claim 7, **characterized in that**
- the Retrieval-Augmented Generation (9) is trained by adding unique combinations between conditions of the peristomal skin and corresponding best suited ostomy care products (1, 2, 3).

9. Method according to claim 2 and one of claims 5 to 8, **characterized in that**
- the Large Language Model (8) processes the collected overall patient status and the information about the ostomy type.

10. Method according to one of the preceding claims, **characterized by** the steps:
- automatically finding recommendations regarding peristomal skin treatments based on the analysed image data (4), and
- outputting the recommendations.

11. System for automatically selecting an ostomy care product from a given set of ostomy care products (1, 2, 3), comprising
- a camera (10) capturing image data (4) of peristomal skin (5) of a patient having a stoma (6),
- processing means (11) for automatically analysing the image data (4) and automatically selecting the ostomy care product from the given set of ostomy care products (1, 2, 3) based on the analysed image data (4), and
- outputting means (12) for outputting information about the selected ostomy care product.
